Europäisches Patentamt

European Patent Office

Office européen des brevets

(19)

(11) **EP 0 513 564 B1**

(12) **EUROPÄISCHE PATENTSCHRIFT**

(45) Veröffentlichungstag und Bekanntmachung des Hinweises auf die Patenterteilung:
**22.12.1999 Patentblatt 1999/51**

(51) Int Cl.6: **G01N 33/84**, G01N 9/00
// G01N33/52

(21) Anmeldenummer: **92106828.4**

(22) Anmeldetag: **22.04.1992**

(54) **Reagenz zur Bestimmung der Ionenstärke bzw. des spezifischen Gewichtes von wässrigen Flüssigkeiten und Verfahren**

Reagent and method to measure the ionic strength or specific gravity of aqueous fluids

Réactif et méthode de détermination de la force ionique ou du poids spécifique d'un liquide aqueux

(84) Benannte Vertragsstaaten:
**AT BE CH DE DK ES FR GB IT LI LU NL PT SE**

(30) Priorität: **17.05.1991 DE 4116108**

(43) Veröffentlichungstag der Anmeldung:
**19.11.1992 Patentblatt 1992/47**

(73) Patentinhaber: **Dade Behring Marburg GmbH**
**35001 Marburg (DE)**

(72) Erfinder: **Habenstein, Klaus**
**W-3552 Wetter (DE)**

(56) Entgegenhaltungen:
EP-A- 0 114 316          EP-A- 0 349 934
EP-A- 0 418 169          CH-A- 456 988
US-A- 4 015 462

**Beschreibung**

Gegenstand der Erfindung ist ein Verfahren zur Bestimmung der Ionenstärke bzw. des spezifischen Gewichtes von wäßrigen Flüssigkeiten sowie ein Reagenz hierfür, wobei die Zusammensetzung des Reagenz so ist, daß die die Ionenstärke anzeigende Farbveränderung im wesentlichen direkt von dem spezifischen Gewicht der zu bestimmenden Flüssigkeit abhängig ist und nicht von einer pH Verschiebung.

Das spezifische Gewicht einer Flüssigkeit kann auf unterschiedlichen Wegen mit verschiedenen apparativen Hilfsmitteln bestimmt werden, beispielsweise mit Aräometern, Pyknometern, Refraktometern. Die erreichbaren Meßgenauigkeiten sind zwar durchweg gut, es sind jedoch mitunter aufwendige Kalibrierungs- und Temperierungs- oder Reinigungsarbeiten mit diesen gerätetechnischen Meßverfahren verbunden. Insbesondere für Serien- oder Reihenuntersuchungen sind solche Verfahren daher nur mit hohem apparativem Aufwand einsetzbar und automatisierbar. Gerade in der klin.-chem. Urinuntersuchung spielt die Frage der Automatisierung und Testvereinfachung im Hinblick auf Kostendegressionen eine besondere Rolle. Hier wurde deshalb schon seit langem mit der Entwicklung der sogenannten Trockenchemie, speziell in der Ausführungsform der Teststreifen, ein zukunftsweisender Weg für die Entwicklungsrichtung der gesamten klinischen Chemie beschritten.

Ein besonderes Problem stellte die Bestimmung des spezifischen Gewichtes von Urin für die Testentwickler in der "Trockenchemie" dar, weil sämtliche bis dato bekannten Testverfahren nicht auf Teststreifen übertragbar waren. Hier kam den Entwicklungschemikern der Umstand zugute, daß der klinische Chemiker in erster Linie an einer Aussage zur Konzentration der harnpflichtigen Substanzen im Urin interessiert ist. Die Dichte wird von ihm deshalb bestimmt, weil sie im wesentlichen mit der gesuchten Stoffkonzentration korreliert.

Unter den harnpflichtigen Substanzen nehmen nun wiederum die Salze eine herausragende Stellung ein. Es lag daher nahe, die Salzkonzentration über eine der kolligativen Eigenschaften zu bestimmen.

Ein Verfahren zur Bestimmung des spezifischen Gewichts bzw. der Ionenstärke auf einem Teststreifen wird in der US-A-4,318,709 und der korrespondierenden DE-A-29 44 980 beschrieben. Darin wird die Probe mit einem Reagenz ver-20 setzt, welches ein schwach saures oder schwach basisches, zu wenigstens 50 % neutralisiertes Polyelektrolytpolymer und einen Indikator enthält. Das zugrundeliegende Prinzip ist die Proportionalität zwischen dem spezifischen Gewicht der Ionenstärke einer wäßrigen Lösung.

Als Polyelektrolytpolymere werden dort Polymere mit ionischen Gruppen bezeichnet. Beispielhaft genannt sind Polyacrylsäure oder Polyvinylamin.

In der EP-A- 0 114 316 werden Reagenzien und Verfahren zur Bestimmung der Ionenstärke beschrieben, die kurzkettige Ammoniumsalze als Gegenionen zu den genannten Polyelektrolyten und Bromthymolblau entwickeln; gemessen wird die durch die Salze bedingte pH-Verschiebung.

Während die Übertragung des Osmose-Meßprinzips (z. B. US 4,015,462) auf Teststreifen offensichtlich technische Schwierigkeiten beinhaltet, konnte die ionenverursachte pH-Änderung in einem Puffersystem recht einfach mit einem Teststreifen gemessen werden. Das Puffersystem enthielt komplexierende Puffersubstanzen und ionenaustauschende Polyelektrolyte. Wie aus den Beispielen der EP 0 023 631 hervorgeht, ist das wesentliche Element zur Erzielung einer pH-Änderung mit steigender Ionenstärke die Art und der pH-Wert der Puffersubstanz.

Aus der EP 0 349 934 ist z. B. ein Verfahren bekannt, das ohne Verwendung von Polyelektrolytpolymeren mindestens eine pH-Puffersubstanz und/oder einen Komplexbildner sowie als Indikator einen pH Indikator enthält.

Da die bisher bekannten Verfahren z. B. EP 349 934 und EP 114 315 eine durch Salze bedingte pH Verschiebung messen, können pH Schwankungen der Urine nur unzureichend durch entsprechende Abpufferung des Teststreifen ausgeglichen werden.

Die bisher bekannten Verfahren haben auch den Nachteil, daß die einmal entstandene Farbe sich mit der Zeit verändert sowie, daß die Teststreifen "ausbluten". Das hat zur Folge, daß sich der Wert, den man für das spezifische Gewicht nach einer Minute abliest, sich von dem Wert, den man nach zwei oder gar fünf Minuten abliest, stark unterscheiden kann.

Wenn der Test also von nicht speziell dafür ausgebildeten Personen, z. B. einem beliebigen Patienten, ausgeführt wird und die Auswertung nicht immer zur gleichen, vom Hersteller des Streifens vorgegebenen Zeit vorgenommen wird, kann das zu folgenschweren Fehlinterpretationen des Gesundheitszustandes führen.

Die Verwendung von Komplexbildnern hat darüberhinaus noch den Nachteil, daß das angezeigte Ergebnis wesentlich von der Zusammensetzung der Ionen, speziell der Metallionen, in der Probe abhängt, da die Affinität der Komplexbildner für verschiedene Ionen unterschiedlich ist. Diese Zusammensetzung ist jedoch auch in Normalurinen starken Schwankungen ausgesetzt.

Der vorliegenden Erfindung lag daher die Aufgabe zugrunde, ein Verfahren zur Bestimmung der Ionenstärke bzw. des spezifischen Gewichts von wäßrigen Flüssigkeiten zu entwickeln, das ein konstantes Farbsignal entwickelt, ionenunabhängig ist und das ein Ausbluten der entsprechend dem Verfahren hergestellten Teststreifen verhindert.

Überraschenderweise wurde gefunden, daß durch Zusatz von Detergenzien zu den dem Fachmann an sich als pH-Indikatoren bekannten und verwendeten Substanzen und Puffersalzen Reagenzien erhalten werden, die das spezifische Gewicht nicht über den Umweg einer pH Messung, sondern direkt als Farbänderung, die durch Ionenkonzentration bewirkt wird, bestimmen (siehe Tab. 1) und aufgrund ihrer außerordentlichen Farbbrillanz eine erheblich höhere Sensitivität besaßen, als die bisher bekannten und darüberhinaus eine große Farbkonstanz und Ausblutechtheit zeigten.

Ein Gegenstand der Erfindung ist somit ein gepuffertes Reagenz zur Bestimmung der Ionenstärke bzw. des spezifischen Gewichtes einer wäßrigen Flüssigkeit mit Hilfe eines Farbindikators, das mindestens ein Detergenz und eine an sich als pH-Indikator bekannte Substanz als Indikator enthält und dessen pH-Verschiebung bei Probenzugabe geringer ist, als es der beobachtbaren Farbverschiebung entspricht.

Das Reagenz enthält als Indikator vorzugsweise Thymolblau oder Bromthymolblau.

Als Detergenz wird ein quarternäres Ammoniumsals oder eine Mischung von mehreren quarternären Ammoniumsalzen verwendet.

Geeignet ist weiterhin ein zwitterionisches Detergenz oder eine Mischung von zwitterionischen Detergenzien.

Das erfindungsgemäße Reagenz ist vorzugsweise auf einen saugfähigen Träger imprägniert.

Ein Gegenstand der Erfindung ist ferner ein Verfahren zur Bestimmung der Ionenstärke bzw. des spezifischen Gewichts einer wäßrigen Flüssigkeit, das dadurch gekennzeichnet ist, daß zur Bestimmung ein Reagenz wie oben beschrieben verwendet wird.

Ein Gegenstand der Erfindung sind außerdem Teststreifen zur Bestimmung der Ionenstärke oder des spezifischen Gewichts einer wäßrigen Flüssigkeit aus einem Kunststoffstreifen und einem darauf befestigten, mit einem Reagenz imprägnierten, saugfähigen Träger, dadurch gekennzeichnet, daß als Reagenz das oben beschriebene Reagenz dient.

In der wäßrigen Flüssigkeit, deren Ionenstärke, auch Osmolarität genannt bzw. deren spezifisches Gewicht in dem erfindungsgemäßen Verfahren ermittelt wird, können weitere Bestandteile gelöst und/oder nicht gelöst sein. Gelöste Bestandteile sind ionische und nicht-ionische Substanzen. Ungelöste Bestandteile können schwerlösliche chemische Substanzen, aber auch andere Materialien, wie biologische Substanzen, beispielsweise Zellen, sein.

Das erfindungsgemäße Verfahren ist besonders gut für Körperflüssigkeiten, wie Schweiß und Harn, geeignet. Insbesondere hat es sich zur Harnuntersuchung als geeignet erwiesen.

pH-Puffer-Substanzen sind dem Fachmann bekannte Substanzen. Eine pH-Puffer-Substanz ist eine Mischung einer schwachen Säure mit einem praktisch vollständig dissoziierten Salz dieser Säure bzw. eine Mischung einer schwachen Base mit einem praktisch vollständig dissoziierten Salz dieser Base (siehe z. B. Römpps Chemie Lexikon, 7. Auflage, Band 5, Stichwort "Puffer"). Der pH-Wert des Puffers ändert sich bei Zugabe von Säuren oder Basen kaum.

Bevorzugte Puffersubstanzen in dem bevorzugten pH-Bereich von 4 bis 11 sind beispielsweise Puffer, die folgende Ionen enthalten: Phosphat, Borat, Carbonat, Citrat, Diäthylmalonat, Nitrilo-tris-methylenphosphat. Ebenfalls geeignet sind zwitterionische Puffer, wie beispielsweise Glycinpuffer oder 2(N-Morpholino)-äthansulfonsäure (MES). Die pH-Puffersubstanzen werden in solchen Konzentrationen eingesetzt, daß die Konzentration nach Zugabe der Probe 0,005 bis 1,0 mol/l, bevorzugterweise 0,08 bis 0,3 mol/l, ganz bevorzugterweise 0,10 - 0,18 mol/l beträgt.

Detergenzien sind dem Fachmann bekannte Substanzen. Die Begriffe Detergenz und Tenside sind im Sinne dieser Erfindung gleichbedeutend (siehe auch Römpps Chemielexikon, 7. Aufl. Bd. 6, Stichwort "Tenside").

Als Detergenzien sind bevorzugt Verbindungen vom Typ quartärer Ammoniumsalze (Quats) und Detergenzien vom Typ der zwitterionischen Detergenzien. Im erfindungsgemäßen Testmittel werden die Detergenzien bevorzugterweise in Konzentrationen von 0,04 % bis 5 % in der Imprägnierlösung eingesetzt. Die Detergenzien können auch zur Verbesserung der Brillanz von Testmitteln nach dem Stand der Technik verwendet werden.

Bevorzugt ist auch die Verwendung von Detergenzgemischen.

Als Indikatoren für die Bestimmung der Ionenstärke oder des spezifischen Gewichts von Harn sind an sich als pH-Indikatoren für einem pH-Wert von 4 bis 12 bekannte Substanzen geeignet. Besonders gut geeignet sind dabei Thymolblau und Bromthymolblau.

Bevorzugterweise liegt die Konzentration des Indikators nach Zugabe der Probe im Bereich von 0,1 bis 100 mmol/l, besonders bevorzugterweise bei 1 bis 50 mmol/l.

Das erfindungsgemäße Reagenz benötigt keine Zugabe von Polyelektrolytpolymer oder Komplexbildner. Die Zugabe von Detergenzien, die Polyelektrolytpolymere oder Komplexbildner enthalten, zu Reagenzien zur Bestimmung der Ionen- stärke oder des spezifischen Gewichtes, führt hingegen bei diesen Reagenzien zu einer höheren Farbbrillanz und ist daher auch Teil dieser Erfindung.

Das erfindungsgemäße Verfahren kann sowohl in Lösung als auch in einem matrixartigen Trägermaterial ausgeführt werden.

Die bevorzugte Ausführungsform des Verfahrens ist die, daß auf einem Teststreifen das Reagenz zweckmäßig als Überzug auf einen saugfähigen Träger aufgebracht ist. Bevorzugte Träger sind poröse Trägermaterialien oder Vliese. Als Vliese werden papierartig aus Fasern aufgebaute Träger verstanden. Mit Reagenz imprägnierte saugfähige Träger werden bevorzugt hergestellt, indem man das saugfähige Trägermaterial mit einer Lösung des Reagenzes tränkt und trocknet. Dadurch bildet sich auf der ganzen, bevorzugt auch der inneren Oberfläche ein Reagenzfilm. Das Reagenz kann auch mit filmbildenden und quellfähigen Zusatzstoffen auf einen Träger, beispielsweise direkt auf dem Teststreifen, aufgebracht sein. Das Reagenz kann weiterhin übliche Zusatzstoffe, wie beispielsweise Stabilisatoren, Netzmittel oder Quellmittel enthalten.

Bei der bevorzugten Ausführungsform des erfindungsgemäßen Verfahrens weist dieser Teststreifen ein Testfeld auf, welches aus einem mit einer Lösung des Reagenz imprägnierten und getrockneten Vlies besteht. Das Testfeld wird mit dem zu untersuchenden Harn in Kontakt gebracht, beispielsweise durch Eintauchen des Teststreifens in den Harn und Herausnahme, sobald das Testfeld naß ist. Die Farbe, die das Testfeld dann angenommen hat, wird mit der Farbe eines dem Teststreifen beiliegenden Muster verglichen. Jeder Farbe des Musters ist dabei ein Wert für ein spezifisches Gewicht zugeordnet. Das zu bestimmende spezifische Gewicht hat den Wert, der dem Muster mit übereinstimmender Farbe zugeordnet ist.

In einer besonders bevorzugten Ausführungsform ist das Testfeld zur Bestimmung der Ionenstärke oder des spezifischen Gewichtes Bestandteil eines dem Fachmann an sich bekannten Mehrfachstreifens.

Bevorzugt ist die Ablesung und Auswertung des Testfeldes in einem Instrument, wie es z. B. unter dem Namen Rapimat[R] (Behringwerke AG, Marburg, Deutschland) bekannt ist.

Eine bevorzugte Ausführungsform des zum Imprägnieren verwendeten Reagenzes kann beispielsweise folgende Zusammensetzung haben:

Puffer: Phosphatpuffer pH 7 - 8 bevorzugterweise 7,5 in einer Endkonzentration von 80 - 300 mmol/l.

Detergenz: Zwitterionisches Detergenz, bevorzugterweise Benzethoniumchlorid 0,2 - 2 % (w/v), bevorzugterweise 1 %.

Indikator: pH-Indikator mit einem Farbumschlagsbereich von pH 7 bis 9, bevorzugterweise Thymolblau oder Bromthymolblau in einer Konzentration von 1 - 50 mmol/l, bevorzugterweise 5 - 20 mmol/l.

Die Auswertung in erfindungsgemäßen Verfahren kann auf an sich bekannte Art und Weise mittels einer Eichkurve erfolgen. Diese Eichkurve ist im Falle der visuellen Auswertung beispielsweise eine Farbskala.

Die Auswertung einer Bestimmung nach dem erfindungsgemäßen Verfahren ist bereits nach 60 Sek. auswertbar, die entstandene Farbe ist dann über einen Zeitraum von wenigstens 15 Min. stabil, sodaß auch z. B. bei längeren Meßreihen die erfaßten Werte noch direkt vergleichbar sind.

Ansprüche und Beispiele sind Bestandteil der Offenbarung.

Die folgenden Beispiele dienen zur Erläuterung der Erfindung und schränken diese in keine Weise ein.

**Beispiel 1:**

Papier der Fa. Machery + Nagel (N 8.18.) wird mit folgender Lösung imprägniert und getrocknet:

In 90 ml 90 mM Natriumdihydrogenphosphatlösung pH ca. 7,5 wird 1 g Benzethoniumchlorid (Fa. Serva) gelöst. Nach Zugabe von 10 ml einer 1,5 %igen methanolischen Bromthymolblau-Lösung wird mit 1 M Natronlauge auf pH 7,5 eingestellt.

Das so erhaltene Testpapier wird in 5 x 5 mm große Quadrate aufgeschnitten und auf steifer Plastikfolie aufgeklebt. Man erhält auf diese Weise Teststreifen, die nach Eintauchen in verschieden konzentrierte Urine visuell und reflexionsphotometrisch ausgewertet werden können. Die Ergebnisse sind in nachstehender Tabelle aufgeführt:

**A) Visuelle Auswertung:**

Tab. 1

| Kochsalzlösung mit folgendem spez. Gewicht | | | |
|---|---|---|---|
| bidest. Wasser | 1,005 | 1,010 | 1,020 |
| grünblau | bläulichgrün | grün | hellgrün |
| nativer Urin m. Kochsalz folg. spez. Gewichte eingestellt | | | |
| ohne Zusatz 1,018 | 1,024 | 1,030 | 1,040 |
| grün | grün | grünlichgelb | gelb |

**B) Auswertung mit einem Automaten**

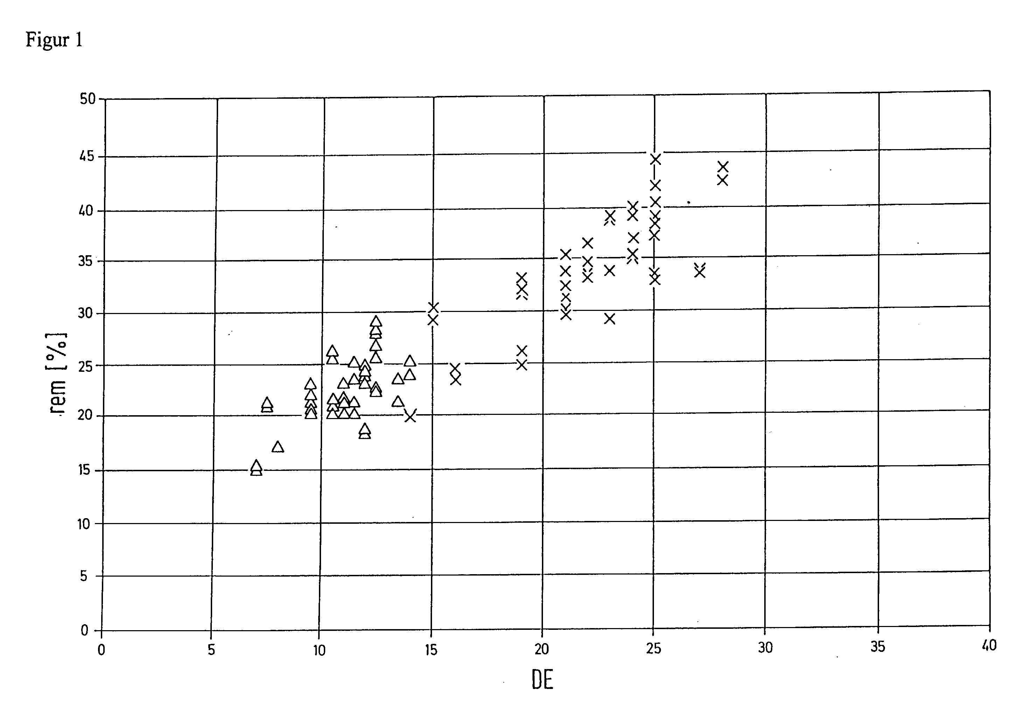

Die wie oben beschrieben hergestellten Teststreifen wurden, nach dem Eintauchen in die zu untersuchenden Urine, vollautomatisch in einem dafür geeigneten Instrument (Rapimat[R], Behringwerke AG, Deutschland) ausgewertet (Fig. 1). Als Urine wurden klinische Urinproben (X) verwendet, die für den unteren Dichtebereich 1+1 mit destilliertem Wasser verdünnt wurden (Δ). Die Dichteeinheit wurde definiert als

$$DE = (\text{gemessene Dichte} - 1{,}000) \times 10^3$$

**Beispiel 2:**

Vergleich mit dem Stand der Technik

Eine Lösung gemäß Beispiel 1 mit 120 mmol/l Puffer, anstelle der 90 mmol/l Puffer, wurde mit Hilfe einer pH-Elektrode vor und nach Zugabe der Probe gemessen. In der Tabelle sind die relativen pH Änderungen im Verhältnis zur jeweiligen Ausgangslösung, die mit einem entsprechenden Volumen an dest. Wasser versetzt wurden, wiedergegeben:

Entsprechende Reagenzien wurden gemäß EP 349 934/Beispiel 1 und EP 114 315/Beispiel 9.1 hergestellt und wie oben beschrieben getestet.

| Probe | Dichte | Beispiel 1 | EP 349 934 | EP 114 315 |
|---|---|---|---|---|
| $H_2O$ | 1,000 | 0,00 | 0,00 | 0,00 |
| 0,4 mol/l NaCl | 1,017 | 0,10 | 0,30 | 0,40 |
| Normalurin | 1,017 | 0,00 | 0,15 | 0,50 |

**Patentansprüche**

**Patentansprüche für folgende Vertragsstaaten : AT, BE, CH, LI, DE, DK, FR, GB, IT, LU, NL, PT, SE**

1. Gepuffertes Reagenz zur Bestimmung der Ionenstärke bzw. des spezifischen Gewichtes einer wäßrigen Flüssigkeit mit Hilfe eines Farbindikators dadurch gekennzeichnet, daß es mindestens ein Detergenz und einen pH-Indikator enthält und daß die pH Verschiebung bei Proben zugabe geringer ist als es der beobachtbaren Farbverschiebung entspricht.

2. Reagenz nach Anspruch 1, dadurch gekennzeichnet, daß der pH-Indikator Thymolblau oder Bromthymolblau ist.

3. Reagenz nach mindestens einem der Ansprüche 1 und 2, dadurch gekennzeichnet, daß das Detergenz ein oder mehrere quarternäre Ammoniumsalze enthält.

4. Reagenz nach mindestens einem der Ansprüche 1 bis 3, dadurch gekennzeichnet, daß das Detergenz ein oder mehrere zwitterionische Detergenzien enthält.

5. Reagenz nach mindestens einem der Ansprüche 1 bis 4, dadurch gekennzeichnet, daß es auf einem saugfähigen Träger imprägniert ist.

6. Verfahren zur Bestimmung der Ionenstärke bzw. des spezifischen Gewichts einer wäßrigen Flüssigkeit, wobei

(i) die Flüssigkeit mit einem gepufferten Reagenz nach Anspruch 1 in Kontakt gebracht wird,

(ii) die Farbverschiebung der resultierenden Mischung bestimmt wird, und

(iii) aus der Farbverschiebung die Ionenstärke bzw. bzw. das spezifische Gewicht ermittelt wird.

7. Verfahren nach Anspruch 6, dadurch gekennzeichnet, daß zur Ermittlung der Ionenstärke bzw. des spezifischen Gewichts eine Eichkurve verwendet wird.

8. Verfahren nach mindestens einem der Ansprüche 6 und 7, dadurch gekennzeichnet, daß das Reagenz auf einen saugfähigen Träger imprägniert ist.

9. Teststreifen zur Bestimmung der Ionenstärke bzw. des spezifischen Gewichts einer wäßrigen Flüssigkeit aus einem Kunststoffstreifen und einem darauf befestigten, mit einem gepufferten Reagenz nach Anspruch 1 imprägnierten, saugfähigen Träger.

**Patentansprüche für folgenden Vertragsstaat : ES**

1. Verfahren zur Bestimmung der Ionenstärke bzw. des spezifischen Gewichts einer wäßrigen Flüssigkeit, wobei

   (i) die Flüssigkeit mit einem gepufferten Reagenz, das mindestens ein Detergenz und einen pH-Indikator enthält und die pH Verschiebung bei Probenzugabe geringer ist als es der beobachtbaren Farbverschiebung entspricht, in Kontakt gebracht wird,

   (ii) die Farbverschiebung der resultierenden Mischung bestimmt wird, und

   (iii) aus der Farbverschiebung die Ionenstärke bzw. bzw. das spezifische Gewicht ermittelt wird.

2. Verfahren nach Anspruch 1, dadurch gekennzeichnet, daß der pH-Indikator Thymolblau oder Bromthymolblau ist.

3. Verfahren nach mindestens einem der Ansprüche 1 und 2, dadurch gekennzeichnet, daß das Detergenz ein oder mehrere quarternäre Ammoniumsalze enthält.

4. Verfahren nach mindestens einem der Ansprüche 1 bis 3, dadurch gekennzeichnet, daß das Detergenz ein oder mehrere zwitterionische Detergenzien enthält.

5. Verfahren nach mindestens einem der Ansprüche 1 bis 4, dadurch gekennzeichnet, daß das Reagenz auf einem saugfähigen Träger imprägniert ist.

6. Verfahren nach Anspruch 1, dadurch gekennzeichnet, daß zur Ermittlung der Ionenstärke bzw. des spezifischen Gewichts eine Eichkurve verwendet wird.

7. Verfahren nach mindestens einem der Ansprüche 1 und 6, dadurch gekennzeichnet, daß das Reagenz auf einen saugfähigen Träger imprägniert ist.

**Claims**

**Claims for the following Contracting States : AT, BE, CH, LI, DE, DK, FR, GB, IT, LU, NL, PT, SE**

1. A buffered reagent for determining the ionic strength and/or the specific weight of an aqueous liquid with the aid of a color indicator, which reagent contains at least one detergent and one pH indicator and wherein the pH shift on sample addition is lower than corresponds to the observable color shift.

2. The reagent as claimed in claim 1, wherein the pH indicator is Thymol Blue or Bromothymol Blue.

3. The reagent as claimed in at least one of claims 1 and 2, wherein the detergent contains one or more quaternary ammonium salts.

4. The reagent as claimed in at least one of claims 1 to 3, wherein the detergent contains one or more zwitterionic detergents.

**5.** The reagent as claimed in at least one of claims 1 to 4, which is impregnated onto an absorbent support.

**6.** A method for determining the ionic strength and/or the specific weight of an aqueous liquid, where

(i) the liquid is brought into contact with a buffered reagent as claimed in claim 1,

(ii) the color shift of the resulting mixture is determined, and

(iii) the ionic strength and/or the specific weight is determined from the color shift.

**7.** The method as claimed in claim 6, wherein a calibration curve is used for determining the ionic strength and/or the specific weight.

**8.** The method as claimed in at least one of claims 6 and 7, wherein the reagent is impregnated onto an absorbent support.

**9.** A test strip for determining the ionic strength and/or the specific weight of an aqueous liquid from a plastic strip and an absorbent support attached thereto and impregnated with a buffered reagent as claimed in claim 1.

**Claims for the following Contracting State : ES**

**1.** A method for determining the ionic strength and/or the specific weight of an aqueous liquid, where

(i) the liquid is brought into contact with a buffered reagent which contains at least one detergent and one pH indicator and the pH shift on sample addition is lower than corresponds to the observable color shift,

(ii) the color shift of the resulting mixture is determined, and

(iii) the ionic strength and/or the specific weight is determined from the color shift.

**2.** The method as claimed in claim 1, wherein the pH indicator is Thymol Blue or Bromothymol Blue.

**3.** The method as claimed in at least one of claims 1 and 2, wherein the detergent contains one or more quaternary ammonium salts.

**4.** The method as claimed in at least one of claims 1 to 3, wherein the detergent contains one or more zwitterionic detergents.

**5.** The method as claimed in at least one of claims 1 to 4, wherein the reagent is impregnated onto an absorbent support.

**6.** The method as claimed in claim 1, wherein a calibration curve is used for determining the ionic strength and/or the specific weight.

**7.** The method as claimed in at least one of claims 1 and 6, wherein the reagent is impregnated onto an absorbent support.

**Revendications**

**Revendications pour les Etats contractants suivants : AT, BE, CH, LI, DE, DK, FR, GB, IT, LU, NL, PT, SE**

**1.** Réactif tamponné, pour la détermination de la force ionique ou du poids spécifique d'un liquide aqueux à l'aide d'un indicateur coloré, caractérisé en ce qu'il contient au moins un détergent et un indicateur de pH et en ce que la variation du pH lors d'addition d'échantillon est plus faible que ce qui correspond à la variation de coloration observable.

2. Réactif selon la revendication 1, caractérisé en ce que l'indicateur de pH est le bleu de thymol ou le bleu de bromothymol.

3. Réactif selon au moins l'une des revendications 1 et 2, caractérisé en ce que le détergent contient un ou plusieurs sels d'ammonium quaternaire.

4. Réactif selon au moins l'une des revendications 1 à 3, caractérisé en ce que le détergent contient un ou plusieurs détergents dipolaires.

5. Réactif selon au moins l'une des revendications 1 à 4, caractérisé en ce qu'il est appliqué par imprégnation sur un support absorbant.

6. Procédé pour la détermination de la force ionique ou du poids spécifique d'un liquide aqueux, dans lequel

   (I) on met le liquide en contact avec un réactif tamponné selon la revendication 1,
   (II) on détermine la variation de coloration du mélange résultant, et
   (III) d'après la variation de coloration, on détermine la force ionique ou le poids spécifique.

7. Procédé selon la revendication 6, caractérisé en ce que, pour la détermination de la force ionique ou du poids spécifique, on utilise une courbe d'étalonnage.

8. Procédé selon au moins l'une des revendications 6 et 7, caractérisé en ce que le réactif est appliqué par imprégnation sur un support absorbant.

9. Bandelette réactive pour la détermination de la force ionique ou du poids spécifique d'un liquide aqueux, constituée d'une bande de matière plastique et d'un support absorbant, appliqué sur celle-ci, imprégné avec un réactif tamponné conforme à la revendication 1.

**Revendications pour l'Etat contractant suivant : ES**

1. Procédé pour la détermination de la force ionique ou du poids spécifique d'un liquide aqueux, dans lequel

   (I) on met le liquide en contact avec un réactif tamponné qui contient au moins un détergent et un indicateur de pH et la variation du pH lors d'addition d'échantillon est plus faible que ce qui correspond à la variation de coloration observable,
   (II) on détermine la variation de coloration du mélange résultant, et
   (III) d'après la variation de coloration, on détermine la force ionique ou le poids spécifique.

2. Procédé selon la revendication 1, caractérisé en ce que l'indicateur de pH est le bleu de thymol ou le bleu de bromothymol.

3. Procédé selon au moins l'une des revendications 1 et 2, caractérisé en ce que le détergent contient un ou plusieurs sels d'ammonium quaternaire.

4. Procédé selon au moins l'une des revendications 1 à 3, caractérisé en ce que le détergent contient un ou plusieurs détergents dipolaires.

5. Procédé selon au moins l'une des revendications 1 à 4, caractérisé en ce que le réactif est appliqué par imprégnation sur un support absorbant.

6. Procédé selon la revendication 1, caractérisé en ce que, pour la détermination de la force ionique ou du poids spécifique, on utilise une courbe d'étalonnage.

7. Procédé selon au moins l'une des revendications 1 et 6, caractérisé en ce que le réactif est appliqué par imprégnation sur un support absorbant.

Figur 1

EP 0 513 564 B1